# FASCICULE DE BREVET EUROPEEN

(11) **EP 1 649 902 B1**
(45) Date de publication et mention de la délivrance du brevet: **11.07.2012**
(21) Numéro de dépôt: 05292000.6
(22) Date de dépôt: 27.09.2005
(51) Int. Cl.: A61Q 17/04, A61K 8/37, A61K 8/35, A61K 8/31, A61K 8/40, A61K 8/49

(54) **Compositon photoprotectrice contenant un dérivé de dibenzoylméthane, un dérivé arylalkyl benzoate et un composé susceptible d'accepter l'énergie de niveau excité triplet dudit dérivé de dibenzoylméthane; procédé de photostabilisation**
Lichtschutzzubereitung enthaltend ein Dibenzoylmethane, ein Arylalkylbenzoat und eine Substanz die Energie vom angeregten Tripletzustand des Dibenzoylmethans aufnehmen kann; Verfahren zur Fotostabilisierung
Light screening composition comprising a dibenzoylmethane derivative, an arylalkyl benzoat derivative and a compound capable of accepting energie from the excited triplet state of dibenzoylmethane; process of photostabilisation

(30) Priorité: 19.10.2004 FR 0452370
(43) Date de publication de la demande: 26.04.2006
(73) Titulaire: L'Oréal, 75008 Paris (FR)
(72) Inventeur: Candau Didier, 91570 Bievres (FR)
(74) Mandataire: Miszputen, Laurent

(56) Documents cités:
- WO-A-03/007906
- WO-A-03/103622
- FR-A- 2 800 991
- US-A- 5 993 789
- US-A1- 2004 057 912
- ISP: "x-tend 226, A NOVEL ESTER WITH HIGH SOLUBILIZING CAPACITY" ISP PRODUCT INFORMATION, août 2003 (2003-08), XP002320817

## Description

La présente invention est relative à une composition photoprotectrice à usage contenant un dérivé de dibenzoylméthane, un dérivé arylalkyl benzoate particulier et un composé susceptible d'accepter l'énergie de niveau excité triplet dudit dérivé de dibenzoylméthane

La présente invention est également relative à un procédé de photostabilisation vis-à-vis du rayonnement UV d'au moins un dérivé du dibenzoylméthane par l'association d'un dérivé arylalkyl benzoate et d'un composé susceptible d'accepter l'énergie de niveau excité triplet dudit dérivé de dibenzoylméthane.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Dans le but d'assurer une protection de la peau et des matières kératiniques contre le rayonnement UV, on utilise généralement des compositions antisolaires comprenant des filtres organiques, actifs dans l'UV-A et actifs dans l'UV-B. La majorité de ces filtres est liposoluble.

A cet égard, une famille de filtres UV-A particulièrement intéressante est actuellement constituée par les dérivés du dibenzoylméthane, et notamment le 4-ter-butyl-4'-méthoxydibenzoyl méthane, qui sont liposolubles et présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et FR-A-2440933, ainsi que dans la demande de brevet européen EP-A-0114607 ; le 4-ter-butyl- 4'-méthoxydibenzoyl méthane est par ailleurs actuellement proposé à la vente sous la dénomination commerciale de « PARSOL 1789 » par la Société ROCHE VITAMINS.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

Dans l'état de la technique, pour résoudre ce problème technique, on a déjà proposé d'associer aux dérivés de dibenzoylméthane des composés susceptibles d'accepter l'énergie de niveau excité triplet dudit dérivé de dibenzoylméthane. C'est le cas par exemple des dérivés de naphtalène brevets US 5993789, US6113931, US6126925, US 6284916 . C'est le cas également des dérivés de fluorène tels que décrits dans les demandes de brevet US200400579912, US200400579914, US200400579916, US200406272. Cependant, la stabilité photochimique des dérivés du dibenzoylméthane obtenue avec ces composés n'est pas encore pleinement satisfaisante.

Or, la Demanderesse vient maintenant de découvrir, de façon surprenante, qu'en ajoutant aux dérivés du dibenzoylméthane mentionnés ci-dessus l'association d'un composé arylalkyl benzoate particulier et d'au moins un composé susceptible d'accepter l'énergie de niveau excité triplet dudit dérivé de dibenzoylméthane, il était possible d'améliorer de manière substantielle et remarquable, la stabilité photochimique (ou photostabilité) de ces mêmes dérivés du dibenzoylméthane.

Cette découverte, essentielle, est à la base de la présente invention. Ainsi, conformément à l'un des objets de la présente invention, il est maintenant proposé une composition cosmétique ou dermatologique, à usage topique, caractérisée par le fait qu'elle comprend au moins, dans un support cosmétiquement acceptable :
(a) au moins un filtre UV du type dérivé du dibenzoylméthane et
(b) le 2-phenylethyl benzoate de formule:
(c) au moins un composé susceptible d'accepter l'énergie de niveau excité triplet dudit dérivé de dibenzoylméthane choisi parmi
   - les dérivés de 4-hydroxy-benzylidene malonate ou les dérivés de 4-hydroxy-cinnamate
   - les sels de pipéridinol ;
   - leurs mélanges.

La présente invention a également pour objet un procédé pour améliorer la stabilité d'au moins un dérivé du dibenzoylméthane vis-à-vis du rayonnement UV consistant à ajouter audit dérivé de dibenzoylméthane l'association du 2-phenylethyl benzoate et d'au moins un composé susceptible d'accepter l'énergie de niveau excité triplet dudit dérivé de dibenzoylméthane tel que défini précédemment.

La présente invention a également enfin pour objet l'utilisation de l'association du 2-phenylethyl benzoate et d'au moins un composé susceptible d'accepter l'énergie de niveau excité triplet d'un dérivé de dibenzoylméthane dans une composition cosmétique ou dermatologique comprenant au moins un dérivé du dibenzoylméthane dans le but de d'améliorer la stabilité vis-à-vis des rayons UV dudit dérivé de dibenzoylméthane tel que défini précédemment.

D'autres caractéristiques, aspects et avantages de l'invention apparaîtront à la lecture de la description détaillée qui va suivre.

Parmi les dérivés du dibenzoylméthane, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthxydibenzoylméthane,
- le 2,4-diméthyl-4'-méthoxydibenzoylméthane,
- le 2,6-diméthyl-4-tert-butyl-4'-méthoxydibenzoylméthane.

Parmi les dérivés du dibenzoylméthane mentionnés ci-dessus, on utilisera en particulier le 4-isopropyl-dibenzoylméthane, vendu sous la dénomination de "EUSOLEX 8020" par la Société MERCK, et répondant à la formule suivante :

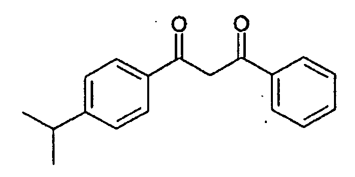

On préfère tout particulièrement mettre en oeuvre le 4-(ter.-butyl) 4'-méthoxy dibenzoylméthane ou Butyl Methoxy Dibenzoylmethane, proposé à la vente sous la dénomination commerciale de "PARSOL 1789" par la Société Roche Vitamins ; ce filtre répond à la formule suivante :

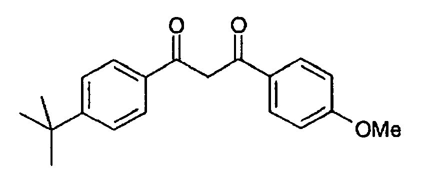

Le ou les dérivés du dibenzoylméthane peuvent être présents dans les compositions conformes à l'invention à des teneurs qui varient de préférence de 0,01 à 10% en poids et plus préférentiellement de 0,1 à 6% en poids par rapport au poids total de la composition.

On utilisera plus particulièrement le 2-éthylphenyle benzoate 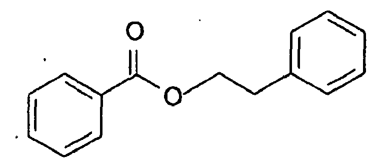 comme le produit commercial X-TEND 226® vendu par la société ISP.

Le 2-éthylphenyle benzoate conformes à l'invention peut être présents dans les compositions conformes à l'invention à des teneurs allant de 0,1 à 40% en poids et plus préférentiellement de 0,1 à 30 % en poids par rapport au poids total de la composition.

Selon la présente invention, on utilise en association avec le 2-éthylphenyle benzoate, un composé susceptible d'accepter l'énergie de niveau excité triplet dudit dérivé de dibenzoylméthane de telle sorte à désactiver les états excités de la molécule dibenzoylméthane excitée sous l'influence des radiations UV et permettre à celle-ci de retrouver son état fondamental.

Selon une forme préférentielle, les composés susceptibles d'accepter l'énergie de niveau excité triplet dudit dérivé de dibenzoylméthane ont une énergie de niveau excité triplet allant de 40 kcal/mol à 70 kcal/mol.

Les énergies de niveau excité triplet peuvent être déterminées par les techniques de perturbation par l'oxygène ou de phosphorescence telles que décrites dans l'article de J. Gonzenbach, T. J. Hill, T.G Truscott « The Triplet Energy Levels in UVA and UVB Sunscreens », J. Photochem. Photobiol. B: Biol, vol 16, pages 337-379 (1992). La technique de pertubation par l'oxygène consiste à mesurer le spectre d'absorption UV d'un composé lorsque celui-ci est placé dans un environnement sous forte pression d'oxygène : ie 2000 psi. Sous ces conditions, les règles de sélection du spin sont perturbées et l'exposition du composé aux UV conduit au niveau excité triplet le plus bas par excitation directe de l'état fondamental. La longueur d'onde λ (en µm) à laquelle la transition s'effectue est utilisée pour calculer l'énergie du niveau triplet en kcal/mol par la formule E= 28.635/λ qui est dérivée de l'équation E = hv où E est l'énergie, h la constante de Planck et v la fréquence de l'onde électromagnétique.

La technique de phosphorescence se base sur le fait que de nombreux composés émettent une phosphorescence lors de la désactivation de leur niveau excité triplet. En mesurant la longueur d'onde à laquelle la phosphorescence intervient les énergies de niveau excité triplet peuvent être calculées comme précédemment. Les énergies de niveau excité triplet peuvent être déterminées en mesurant les spectres de phosphorescence d'échantillons avec un spectrophotomètre équipé d'un accessoire de phosphorescence. De tels niveaux excités triplets ont été largement reportés par exemple dans l'article de A. J. Gordon, R. A. Ford, « The Chemist Companion », John Wiley & Sons, pages 351-355 (1992).

Les composés du niveau excité triplet dudit dérivé de dibenzoylméthane sont par choisis parmi :
- les dérivés de 4-hydroxy-benzylidene malonate ou les dérivés de 4-hydroxy-cinnamate tels que ceux décrits dans la demande de brevet WO03/007906 ;
- les sels de pipéridinol tels que ceux décrits dans la demande de brevet WO03/103622 ;
- leurs mélanges.

Parmi les dérivés de 4-hydroxy-benzylidene malonate ou les dérivés de 4-hydroxy-cinnamate, on utilisera préférentiellement ceux de formule (VI) : 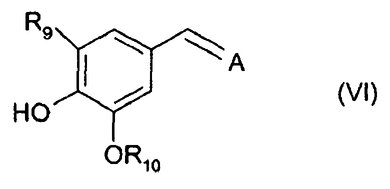 dans laquelle A est un groupe chromophore absorbant les radiations UV comprenant deux groupes monovalents ayant une fonction carbonyle ;
R₉ désigne hydrogène, un radical alkyle en C₁-C₈, linéaire ou ramifié ou un radical alkoxy en C₁-C₈, linéaire ou ramifié.
R₁₀ désigne un radical alkyle en C₁-C₈, linéaire ou ramifié ;

Parmi ces composés, on utilisera plus préférentiellement ceux de formule (VIa) suivante : 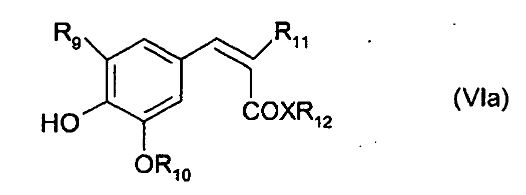 dans laquelle
R₉ désigne hydrogène, un radical alkyle en C₁-C₈, linéaire ou ramifié ; un radical alkoxy en C₁-C₈, linéaire ou ramifié.
R₁₀ désigne hydrogène, un radical alkyle en C₁-C₈, linéaire ou ramifié ;
R₁₁ est choisi parmi -C(O)CH₃ , -CO₂R₁₃, -C(O)NH₂ et -C(O)N(R₁₄)₂ ;
R₁₂ désigne un radical alkyle en C₁-C₃₀, linéaire ou ramifié ;
R₁₃ représente un radical alkyle en C₁-C₂₀, linéaire ou ramifié ;
chaque R₁₄ représente indépendamment un radical alkyle en C₁-C₈, linéaire ou ramifié ;
X désigne O ou NH ;

Parmi ces composés, on utilisera plus préférentiellement ceux de formule (Vlb) ou (VIc) suivante : 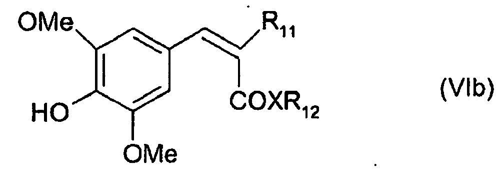 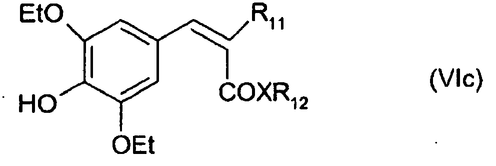 dans lesquelles
R₁₁ désigne -CO₂R₁₃
R₁₂ désigne un alkyle linéaire ou ramifié en C₁-C₈
R₁₃ désigne un alkyle linéaire ou ramifié en C₁-C₈
X désigne O.

On utilisera en particulier le composé Diethylhexyl Syringylidenemalonate (nom INCl) de formule suivante : 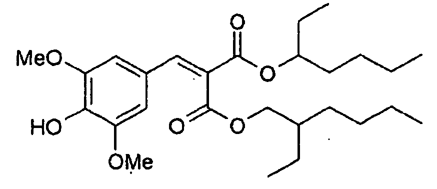 tel que le produit commercial vendu sous la dénomination commerciale de Oxynex ST par la société Merck .

Parmi les sels de pipéridinol conformes à l'invention, on peut utilisera de préférence ceux de formule (XV) suivante : 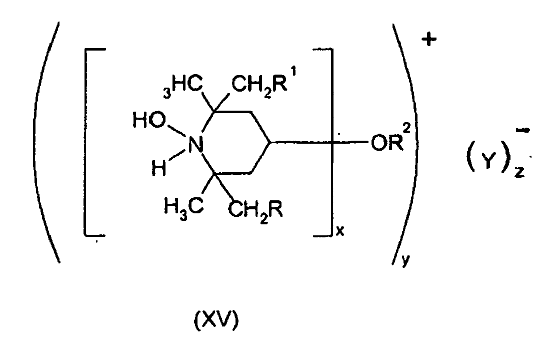 dans laquelle
R¹ désigne hydrogène ou méthyle
x vaut 1 ou 2
1) lorsque x est égal à 1 :
   R² désigne un hydrogène ; un radical alkyle en C₁-C₁₈ ; un radical alcényle en C₂-C₁₈ ; un radical propargyle ; un groupe glycidyle ; un radical alkyle en C₂-C₅₀ interrompu par 1 à 20 atomes d'oxygène ledit alkyle étant substitué par 1 à 10 groupes hydroxyles ou bien à la fois interrompu par lesdits atomes d'oxygène et substitué par lesdits groupes hydroxyles ; un radical alkyle en C₁-C₄ substitué par un groupe carboxy ou un groupe -COOZ où Z est hydrogène, un alkyle en C1-C4, phényle, un alkyle en C₁-C₄ substitué par un groupe (COO ⁻) ₚ M ^{p+} où p est un entier de 1 à 3 et M un ion métallique des groupes 1, 2 et 3 du tableau périodique ou Zn , Cu, Ni ou Co ou bien M est un groupe N ^{p+} (R")₄ où R" est un alkyle en C₁-C₈ ou un benzyle ;
2) lorsque x vaut 2 ,
   R' est un radical alkylène en C₁-C₁₂ ; un radical alcénylène en C₄-C₁₂ ; un groupe xylylène ; un radical alkylène en C₁-C₅₀ interrompu par 1 à 20 atomes d'oxygène ledit alkyle étant substitué par 1 à 10 groupes hydroxyles ou bien à la fois interrompu par lesdits atomes d'oxygène et substitué par lesdits groupes hydroxyles ;
   Y désigne un anion organique ou minéral ;
   la charge totale en cations y étant égal à la charge totale en anions z.

Parmi les anions Y, on peut citer phosphate, phosphonate, carbonate, bicarbonate, nitrate, chlorure, bromure, bisulfite, sulfite, bisulfate, sulfate, borate, formate,acétate, benzoate, citrate, oxalate, tartrate, acrylate, polyacrylate, fumarate, maléate, itaconate, glycolate, gluconate, malate, mandélate, tiglate, ascorbate, polyméthacrylate, un carboxylate de l'acide nitrilotriacétique, l'acide hydroxyéthylènediaminetriacétique, l'acide éthylènediaminetétraacétique, l'acide diéthylènediaminepentaacétique, le diéthylènetriaminepentaméthylènephosphonate, un alkylsulfonate ou un arylsulfonate.

On utilisera en particulier les composés de formule (XV) pour lesquels R¹ et R² désignent l'hydrogène, x = 1 et Y désigne l'anion citrate et encore plus particulièrement le composé Tris(tétraméthylhydroxypipéridinol) citrate de structure : 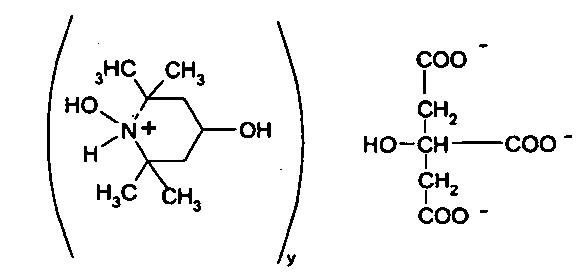 avec y = 3 tel que le produit commercial vendu le nom TINOGUARD Q ou TINOGUARD S-FX par la société CIBA- GEIGY.

Les composés susceptibles d'accepter l'énergie de l'état triplet des dérivés du dibenzoylméthane conformes à l'invention peuvent être présents dans les compositions conformes à l'invention à des teneurs allant de 0,1 à 25% en poids et plus préférentiellement de 0,2 à 10 % en poids en encore plus préférentiellement de 0,2 à 7% en poids par rapport au poids total de la composition.

Selon la présente invention, le mélange photostabilisant dérivé arylalkyl benzoate/composé susceptible d'accepter l'énergie de l'état triplet des dérivés du dibenzoylméthane sera utilisée dans une quantité suffisante permettant d'obtenir une amélioration notable et significative de la photostabilité du dérivé du dibenzoylméthane dans une composition donnée. Cette quantité minimale en agent photostabilisant à mettre en oeuvre peut varier selon la quantité de dibenzoylméthane présent au départ dans la composition et selon la nature du support cosmétiquement acceptable retenu pour la composition. Elle peut être déterminée sans aucune difficulté au moyen d'un test classique de mesure de photostabilité.

Les compositions conformes à l'invention peuvent comporter en plus d'autres agents photoprotecteurs organiques ou inorganiques complémentaires actifs dans l'UVA et/ou l'UVB hydrosolubles ou liposolubles ou bien insolubles dans les solvants cosmétiques couramment utilisés.

Les agents photoprotecteurs organiques complémentaires sont notamment choisis parmi les anthranilates ; les dérivés salicyliques, les dérivés du camphre ; les dérivés de la benzophénone ; les dérivés de β,β-diphénylacrylate ; les dérivés de triazine ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples d'agents photoprotecteurs organiques complémentaires, on peut citer ceux désignés ci-dessous sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés de β,β-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés du phenyl benzotriazole :

Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés de triazine :

- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris-(4'-amino benzalmalonate de düsobutyle)-s- triazine.

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Polyorganosiloxane à fonctions benzalmalonate comme le Polysilicone-15 vendu sous la dénomination commerciale « PARSOL SLX » par HOFFMANN LA ROCHE

### Dérivés de 4,4-diarylbutadiène :

- 1,1-dicarboxy (2,2'-diméthyl-propyl)-4,4-diphénylbutadiène

### Dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V
et leurs mélanges.

Les agents photoprotecteurs organiques complémentaires préférentiels sont choisis parmi
Ethylhexyl Salicylate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Ethylhexyl triazone,
Diethylhexyl Butamido Triazone,
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
et leurs mélanges.

Les agents photoprotecteurs complémentaires inorganiques sont choisis parmi des pigments et plus préférentiellement encore des nanopigments (taille moyenne des particules primaires : généralement entre 5 nm et 100 nm, de préférence entre 10 nm et 50 nm) d'oxydes métalliques traités ou non comme par exemple des nanopigments d'oxyde de titane (amorphe ou cristallisé sous forme rutile et/ou anatase), de fer, de zinc, de zirconium ou de cérium .

Les nanopigments traités sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p 53-64, tels que des aminoacides, de la cire d'abeille, des acides gras, des alcools gras, des tensio-actifs anioniques, des lécithines, des sels de sodium, potassium, zinc, fer, ou aluminium d'acides gras, des alcoxydes métalliques (de titane ou d'aluminium), du polyéthylène, des silicones, des protéines (collagène, élastine) des alcanolamines, des oxydes de silicium, des oxydes métalliques, de l'hexamétaphosphate de sodium, de l'alumine ou de la glycérine.

Les nanopigments traités peuvent être plus particulièrement des oxydes de titane traités par:
- la silice et l'alumine tels que les produits « Microtitanium Dioxide MT 500 SA » et « Microtitanium Dioxide MT 100 SA » de la société TAYCA, et les produits « Tioveil Fin », « Tioveil OP », « Tioveil MOTG » et « Tioveil IPM » de la société TIOXIDE,
- l'alumine et le stéarate d'aluminium tels que le produit « Microtitanium Dioxide MT 100 T » de la société TAYCA,
- l'alumine et le laurate d'aluminium tels que le produit « Microtitanium Dioxide MT 100 S » de la société TAYCA,
- des oxydes de fer et le stéarate de fer tels que le produit « Microtitanium Dioxide MT 100 F » de la société TAYCA,
- la silice, l'alumine et la silicone tels que les produits « Microtitanium Dioxide MT 100 SAS », « Microtitanium Dioxide MT 600 SAS » et « Microtitanium Dioxide MT 500 SAS » de la société TAYCA,
- l'hexamétaphosphate de sodium tels que le produit « Microtitanium Dioxide MT 150 W » de la société TAYCA,
- l'octyltriméthoxysilane tels que le produit « T-805 » de la société DEGUSSA,
- l'alumine et l'acide stéarique tels que le produit « UVT-M160 » de la société KEMIRA,
- l'alumine et la glycérine tels que le produit « UVT-M212» de la société KEMIRA,
- l'alumine et la silicone tels que le produit « UVT-M262» de la société KEMIRA.

D'autres nanopigments d'oxyde de titane traités avec une silicone sont de préférence le TiO₂ traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 CARDRE UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les nanopigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les nanopigments d'oxyde de zinc non enrobés, sont par exemple
- ceux commercialisés sous la dénomination "Z-COTE" par la société SUNSMART ;
- ceux commercialisés sous la dénomination "NANOX" par la société ELEMENTIS ;
- ceux commercialisés sous la dénomination "NANOGARD WCD 2025" par la société NANOPHASE TECHNOLOGIES ;

Les nanopigments d'oxyde de zinc enrobés sont par exemple
- ceux commercialisés sous la dénomination "OXIDE ZINC CS-5" par la société Toshibi (ZnO enrobé par polymethylhydrogenesiloxane) ;
- ceux commercialisés sous la dénomination "NANOGARD ZINC OXIDE FN" par la société NANOPHASE TECHNOLOGIES (en dispersion à 40% dans le Finsolv TN, benzoate d'alcools en C₁₂-C₁₅) ;
- ceux commercialisés sous la dénomination "DAITOPERSION ZN-30" et "DAITOPERSION ZN-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD ULTRAFINE ZNO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "ESCALOL Z100" par la société ISP (ZnO traité alumine et dispersé dans le mélange methoxycinnamate d'ethylhexyle / copolymère PVP-hexadecene / methicone) ;
- ceux commercialisés sous la dénomination "FUJI ZNO-SMS-10" par la société Fuji Pigment (ZnO enrobé silice et polymethylsilsesquioxane) ;
- ceux commercialisés sous la dénomination "NANOX GEL TN" par la société Elementis (ZnO dispersé à 55% dans du benzoate d'alcools en C₁₂-C₁₅ avec polycondensat d'acide hydroxystéarique).

Les nanopigments d'oxyde de cérium non enrobé est vendu sous la dénomination "COLLOIDAL CERIUM OXIDE" par la société RHONE POULENC.

Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les nanopigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les nanopigments peuvent être introduits dans les compositions selon l'invention tels quels ou sous forme de pâte pigmentaire, c'est-à-dire en mélange avec un dispersant, comme décrit par exemple dans le document GB-A-2206339.

Les agents photoprotecteurs additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » ou « Witconol TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les carbopols (carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer /isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryldimethyltauramide) ; les dérivés cellulosiques tels que l'hydroxyéthylcellulose ; les polysaccharides et notamment les gommes telles que la gomme de xanthane ; et leurs mélanges.

Comme épaississants lipophiles, on peut citer les argiles modifiées telles que le l'hectorite et ses dérivés, comme les produits commercialisés sous les noms de Bentone.

Parmi les actifs, on peut citer :
- les agents anti-pollution et/ou agent anti-radicalaire ;
- les agents dépigmentants et/ou des agents pro-pigmentants ;
- les agents anti-glycation ;
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants ;
- les agents tenseurs.
- les agents desquamants ;
- les agents hydratants ;
- les agents anti-inflammatoires ;
- les agents agissant sur le métabolisme énergétique des cellules.
- les agents répulsifs contre les insectes
- les antagonistes de substances P ou de CRGP.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile. Elles peuvent se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostearate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition autoémulsionnante, comme décrit par exemple dans le document WO-A-92/06778.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin et des produits de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les exemples concrets, mais nullement limitatifs, illustrant l'invention, vont maintenant être donnés.

### Exemples 1 et 2 de composition

On a réalisé les compositions suivantes ; les quantités étant exprimées en % en poids.

| COMPOSITION | FORMULE 1 | FORMULE 2 |
|---|---|---|
| **Poly Dimethylsiloxane** | **0,5** | **0,5** |
| **Conservateurs** | **1** | **1** |
| **Acide Stéarique** | **1,5** | **1,5** |
| **Mélange mono-stéarate de glycéryle / stéarate de PEG (100 OE)** | **1** | **1** |
| **Mélange de cétylstéaryl glucoside et d'alcools cétylique, stéarylique** | **2** | **2** |
| **Alcool cétylique** | **0,5** | **0,5** |
| **4-Tertiobutyl-4'-Methoxy-Dibenzoylmethane** | **2** | **2** |
| **Benzoate d'alcools en C₁₂-C₁₃** | **10** | **5** |
| **Benzoic acid, 2-phenyl ethyl ester** | **10** | **20** |
| **Diethylhexyl Syringylidenemalonate (OXYNEX ST de MERCK)** | **0,51** | - |
| **Tris(tétraméthylhydroxypipéridinol) citrate (TINOGUARD Q de CIBA-GEYGY)** | | **1 (MA)*** |
| **Eau dé-ionisée** | **QSP 100** | **QSP 100** |
| **Complexant** | **0,1** | **0,1** |
| **Glycérol** | **5** | **5** |
| **Gomme dexanthane** | **0,2** | **0,2** |
| **Phosphate de mono cétyle** | **1** | **1** |
| **PHASE C** | | |
| **Iso-Hexadecane** | **1** | **1** |
| **Copolymère acide de stéaryle acrylique/méthacrylate de stéaryle** | **0,2** | **0,2** |
| **Triethanolamine** | **QSP pH** | **QSP pH** |

| | | |
|---|---|---|
| *MA = matière active | | |

### MODE OPERATOIRE :

On chauffe la phase aqueuse (Phase B) contenant l'ensemble de ses ingrédients à 80°C au bain marie. On chauffe la phase grasse (Phase A) contenant l'ensemble de ses ingrédients à 80°C au bain marie. On émulsionne A dans B sous agitation de type rotor-stator (appareil de la société Moritz). On Incorpore la Phase C et on laisse revenir à température ambiante sous agitation modérée. On Introduit la triéthanolamine de façon à ajuster le pH à la valeur désirée en fin de fabrication.

## Revendications

1. Composition photoprotectrice, à usage topique, **caractérisée par le fait qu'**elle comprend au moins, dans un support cosmétiquement acceptable :
(a) au moins un filtre UV du type dérivé du dibenzoylméthane et
(b) le 2-phenylethyl benzoate de formule : 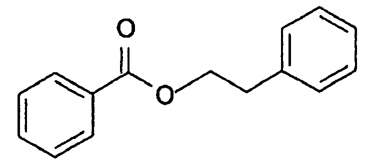
(c) au moins un composé susceptible d'accepter l'énergie de niveau excité triplet dudit dérivé de dibenzoylméthane choisi parmi
(i) les dérivés de 4-hydroxy-benzylidene malonate ou les dérivés de 4-hydroxy-cinnamate ;
(ii) les sels de pipéridinol
(iii) leurs mélanges.

2. Composition selon la revendication 1, où le dérivé du dibenzoylméthane est choisi parmi :
- le 2-méthyldibenzoylméthane,
- le 4-méthyldibenzoylméthane,
- le 4-isopropyldibenzoylméthane.
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthxydibenzoylméthane,
- le 2,4-diméthyl-4'-méthoxydibenzoylméthane,
- le 2,6-diméthyl-4-tert-butyl-4'-méthoxydibenzoylméthane.

3. Composition selon la revendication 1 ou 2, où le dérivé du dibenzoylméthane est le 4-(tert.-butyl)4'-méthoxydibenzoylméthane ou Butyl Methoxy Dibenzoylmethane.

4. Composition selon l'une quelconque des revendications 1 à 3, où le 2-phenylethyl benzoate est présent à des teneurs allant de 0,1 à 40% en poids et plus préférentiellement de 1 à 30 % en poids par rapport au poids total de la composition.

5. Composition selon l'une quelconque des revendications 1 à 4, où le ou les dérivés du dibenzoylméthane sont présents à des teneurs allant de 0,01 à 10% en poids et plus préférentiellement de 0,1 à 6% en poids par rapport au poids total de la composition.

6. Composition selon l'une quelconque des revendications 1 à 5, où les dérivés de 4-hydroxy-benzylidene malonate ou les dérivés de 4-hydroxy-cinnamate sont choisis parmi ceux de formule (VI) : 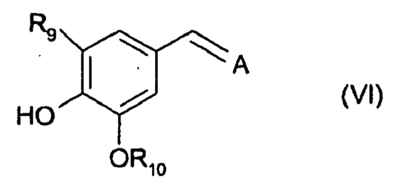 dans laquelle A est un groupe chromophore absorbant les radiations UV comprenant deux groupes monovalents ayant une fonction carbonyle ;
R₉ désigne hydrogène, un radical alkyle en C₁-C₈, linéaire ou ramifié ou un radical alkoxy en C₁-C₈, linéaire ou ramifié.
R₁₀ désigne un radical alkyle en C₁-C₈, linéaire ou ramifié ;

7. Composition selon la revendication 6, où les composés de formule (VI) sont choisis parmi ceux de formule (VIa) suivante : 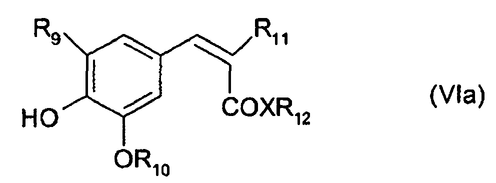 dans laquelle R₁₁ est choisi parmi -C(O)CH₃ , -CO₂R₁₃, -C(O)NH₂ et -C(O)N(R₁₄)₂ ;
X désigne O ou NH ;
R₁₂ désigne un radical alkyle en C₁-C₃₀, linéaire ou ramifié ;
R₁₃ représente un radical alkyle en C₁-C₂₀, linéaire ou ramifié ;
chaque R₁₄ représente indépendamment un radical alkyle en C₁-C₈, linéaire ou ramifié;
R₉ désigne hydrogène, un radical alkyle en C₁-C₈, linéaire ou ramifié ; un radical alkoxy en C₁-C₈, linéaire ou ramifié.
R₁₀ désigne hydrogène, un radical alkyle en C₁-C₈, linéaire ou ramifié ;

8. Composition selon la revendication 6, où les composés de formule (VI) sont choisis parmi ceux de formule (Vlb) ou (VIc) suivante : 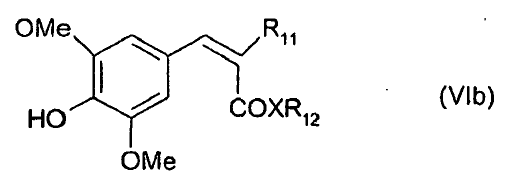 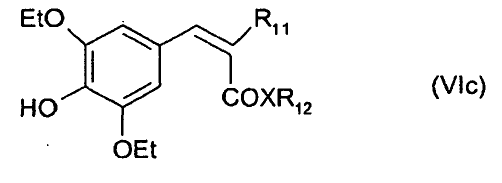 dans lesquelles
R₁₁ désigne -CO₂R₁₃
R₁₂ désigne un alkyle linéaire ou ramifié en C₁-C₈
R₁₃ désigne un alkyle linéaire ou ramifié en C₁-C₈
X désigne O.

9. Composition selon la revendication 8, où le composé de formule (Vlb) est le Diethylhexyl Syringylidenemalonate de formule suivante :

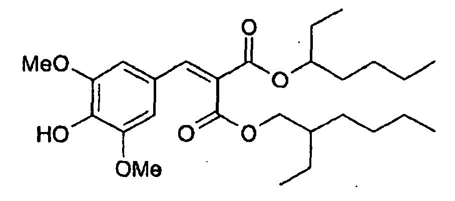

10. Composition selon l'une quelconque des revendications 1 à 5, où les sels de pipéridinol sont choisis parmi ceux de formule (XV) suivante : 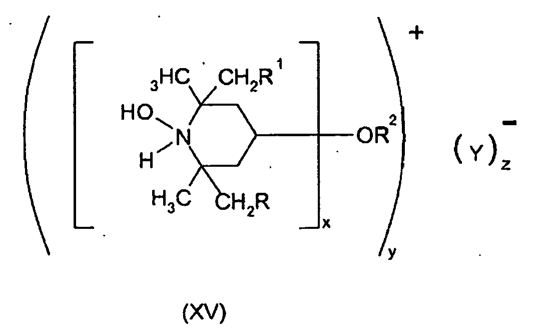 dans laquelle
R¹ désigne hydrogène ou méthyle
x vaut 1 ou 2
1) lorsque x est égal à 1 :
R² désigne un hydrogène ; un radical alkyle en C₁-C₁₈; un radical alcényle en C₂-C₁₈ un radical propargyle ; un groupe glycidyle ; un radical alkyle en C₂-C₅₀ interrompu par 1 à 20 atomes d'oxygène ledit alkyle étant substitué par 1 à 10 groupes hydroxyles ou bien à la fois interrompu par lesdits atomes d'oxygène et substitué par lesdits groupes hydroxyles ; un radical alkyle en C₁-C₄ substitué par un groupe carboxy ou un groupe - COOZ où Z est hydrogène, un alkyle en C1-C4, phényle, un alkyle en C₁-C₄ substitué par un groupe (COO ⁻) ₚM ^{p+} où p est un entier de 1 à 3 et M un ion métallique des groupes 1, 2 et 3 du tableau périodique ou Zn , Cu, Ni ou Co ou bien M est un groupe N ^{p+} (R")₄ où R" est un alkyle en C₁-C₈ ou un benzoyle;
2) lorsque x vaut 2 ,
R' est un radical alkylène en C₁-C₁₂; un radical alcénylène en C₄-C₁₂; un groupe xylylène ; un radical alkylène en C₁-C₅₀ interrompu par 1 à 20 atomes d'oxygène ledit alkyle étant substitué par 1 à 10 groupes hydroxyles ou bien à la fois interrompu par lesdits atomes d'oxygène et substitué par lesdits groupes hydroxyles ;
Y désigne un anion organique ou minéral ;
la charge totale en cations y étant égal à la charge totale en anions z.

11. Composition selon la revendication 10 où les composés de formule (XV) sont choisis parmi ceux pour lesquels R¹ et R² désignent l'hydrogène, x = 1 et Y désigne l'anion citrate.

12. Composition selon la revendication 11 où le composé de formule (XV) est le Tris(tétraméthylhydroxypipéridinol) citrate de structure : 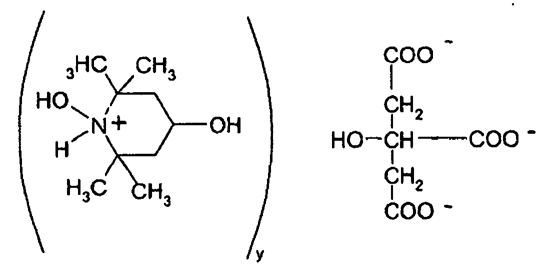 avec y = 3.

13. Composition selon l'une quelconque des revendications 1 à 12, où les composés suspeptibles d'accepter l'énergie de l'état triplet des dérivés du dibenzoylméthane sont présents à des teneurs allant de 0,1 à 25% en poids et plus préférentiellement de 0,2 à 10 % en poids en encore plus péférentiellement de 0,2 à 7% en poids par rapport au poids total de la composition.

14. Composition selon l'une quelconque des revendications 1 à 13, **caractérisée par le fait qu'**elle contient en plus d'autres agents photoprotecteurs organiques ou inorganiques actifs dans l'UV-A et/ou l'UV-B hydrosolubles ou liposolubles ou bien insolubles dans les solvants cosmétiques couramment utilisés.

15. Composition selon la revendication 14, où les agents photoprotecteurs organiques complémentaires sont choisis parmi les anthranilates ; les dérivés salicyliques, les dérivés du camphre ; les dérivés de la benzophénone ; les dérivés de β, β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes et leurs mélanges.

16. Composition selon la revendication 15, **caractérisée par le fait que** le ou les filtres UV organiques sont choisis parmi les composés suivants :
Homosalate
Ethylhexyl Salicylate,
Octocrylene,
Phenylbenzimidazole Sulfonic Acid,
Benzophenone-3,
Benzophenone-4,
Benzophenone-5,
2-(4-dlethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
la 2,4,6-tris-(4'-amino benzalmalonate de dilsobutyle)-s- triazine,
2,4,6-tris(biphenyl-4-yl-1,3,5-triazine
2,4,6-tris(terphenyl)-1,3,5-triazine
Drometrizole Trisiloxane
Polysilicone-15
Dineopentyl 4'methoxybenzalmalonate
1,1-dicarboxy(2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
et leurs mélanges.

17. Composition selon la revendication 14, **caractérisée par le fait que** les agents photoprotecteurs inorganiques complémentaires sont des pigments ou des nanopigments d'oxydes métalliques, traités ou non.

18. Composition selon la revendication 17, **caractérisée par le fait que** lesdits pigments ou nanopigments sont choisis parmi les oxydes de titane, de zinc, de fer, de zirconium, de cérium et leurs mélanges, traités ou non.

19. Composition selon l'une quelconque des revendications 1 à 18, **caractérisée par le fait qu'**elle comprend en outre au moins un agent de bronzage et/ou de brunissage artificiel de la peau.

20. Composition selon l'une quelconque des revendications 1 à 19, **caractérisée par le fait qu'**elle comprend en outre au moins un adjuvant choisi parmi les corps gras, les solvants organiques, les épaississants ioniques ou non ioniques, hydrophiles ou lipophiles, les adoucissants, les humectants, les opaclfiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants

21. Utilisation d'une composition telle que définie dans l'une quelconque des revendications 1 à 20 41 pour la fabrication de produits pour le traitement cosmétique de la peau, des lèvres, des ongles, des cheveux, des cils, sourcils et/ou du cuir chevelu

22. Utilisation d'une composition telle que définie dans l'une quelconque des revendications 1 à 20 44 pour la fabrication de produits de soin de la peau, des lèvres, des ongles, des cheveux et/ou du cuir chevelu.

23. Utilisation d'une composition telle que définie dans l'une quelconque des revendications 1 à 20 pour la fabrication de produits de maquillage.

24. Procédé de photostabilisation vis-à-vis du rayonnement UV d'un dérivé du dibenzoylméthane tel que défini dans l'une quelconque des revendications précédentes par l'association du 2-phenylethyl benzoate et d'un composé susceptible d'accepter l'énergie de niveau excité triplet dudit dérivé de dibenzoylméthane telle que définie dans l'une quelconque des revendications précédentes.

25. Utilisation de l'association du 2-phenylethyl benzoate et d'un composé susceptible d'accepter l'énergie de niveau excité triplet d'un dérivé de dibenzoylméthane tel que définie dans l'une quelconque des revendications précédentes dans une composition cosmétique ou dermatologique comprenant au moins un dérivé du dibenzoylméthane tel que défini dans l'une quelconque des revendications précédentes dans le but de d'améliorer la stabilité vis-à-vis des rayons UV dudit dérivé de dibenzoylméthane.

## Claims

1. Photoprotective composition for topical use, **characterized in that** it comprises, in a cosmetically acceptable support:
(a) at least one UV-screening agent of the dibenzoylmethane derivative type,
(b) 2-phenylethyl benzoate of formula: 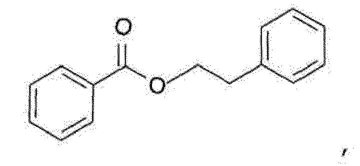 and
(c) at least one compound capable of accepting the excited triplet level energy of the said dibenzoylmethane derivative chosen from
(i) 4-hydroxybenzylidenemalonate derivatives or 4-hydroxycinnamate derivatives;
(ii) piperidinol salts;
(iii) mixtures thereof.

2. Process according to Claim 1, in which the dibenzoylmethane derivative is chosen from:
- 2-methyldibenzoylmethane
- 4-methyldibenzoylmethane
- 4-isopropyldibenzoylmethane
- 4-tert-butyldibenzoylmethane
- 2,4-dimethyldibenzoylmethane
- 2,5-dimethyldibenzoylmethane
- 4,4'-diisopropyldibenzoylmethane
- 4,4'-dimethoxydibenzoylmethane
- 4-tert-butyl-4'-methoxydibenzoylmethane
- 2-methyl-5-isopropyl-4'-methoxydibenzoylmethane
- 2-methyl-5-tert-butyl-4'-methoxydibenzoylmethane
- 2,4-dimethyl-4'-methoxydibenzoylmethane
- 2,6-dimethyl-4-tert-butyl-4'-methoxydibenzoylmethane.

3. Process according to Claim 1 or 2, in which the dibenzoylmethane derivative is 4-(tert-butyl)-4'-methoxydibenzoylmethane or Butyl Methoxy Dibenzoylmethane.

4. Composition according to any one of Claims 1 to 3, in which 2-phenylethyl benzoate is present in contents ranging from 0.1% to 40% by weight and more preferably from 1% to 30% by weight relative to the total weight of the composition.

5. Composition according to any one of Claims 1 to 4, in which the dibenzoylmethane derivative(s) is (are) present in contents ranging from 0.01% to 10% by weight and more preferentially from 0.1% to 6% by weight relative to the total weight of the composition.

6. Composition according to any one of Claims 1 to 5, in which the 4-hydroxybenzylidenemalonate derivatives or the 4-hydroxycinnamate derivatives are chosen from those of formula (VI): 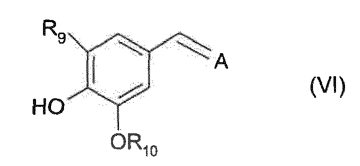 in which A is a chromophoric group that absorbs UV radiation, comprising two monovalent groups containing a carbonyl function;
R₉ denotes hydrogen, a linear or branched C₁-C₈ alkyl radical or a linear or branched C₁-C₈ alkoxy radical,
R₁₀ denotes a linear or branched C₁-C₈ alkyl radical.

7. Composition according to Claim 6, in which the compounds of formule (VI) are chosen from those of formula (VIa) below: 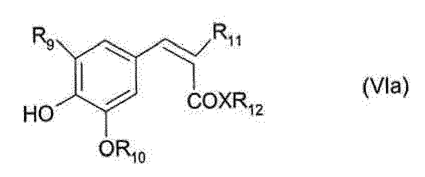 in which
R₁₁ is chosen from -C(O)CH₃, -CO₂R₁₃, -C(O)NH₂, and -C(O)N(R₁₄)₂,
X denotes O or NH,
R₁₂ denotes a linear or branched C₁-C₃₀ alkyl radical,
R₁₃ represents a linear or branched C₁-C₂₀ alkyl radical,
each R₁₄ independently represents a linear or branched C₁-C₈ alkyl radical,
R₉ denotes hydrogen, a linear or branched C₁-C₈ alkyl radical or a linear or branched C₁-C₈ alkoxy radical, R₁₀ denotes hydrogen or a linear or branched C₁-C₈ alkyl radical.

8. Composition according to Claim 6, in which the compounds of formule (VI) are chosen from those of formule (VIb) or (VIc) below: 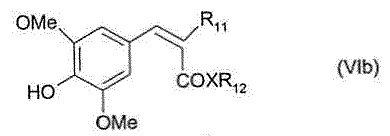 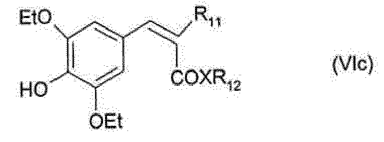 in which
R₁₁ denotes -CO₂R₁₃
R₁₂ denotes a linear or branched C₁-C₈ alkyl
R₁₃ denotes a linear or branched C₁-C₈ alkyl
X denotes O.

9. Composition according to Claim 8, in which the compound of formula (VIb) is diethylhexyl syringylidenemalonate having the following formula:

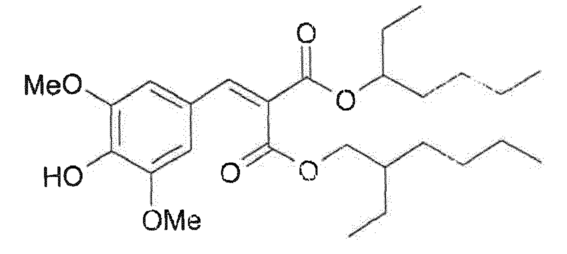

10. Composition according to any one of Claims 1 to 5, in which the piperidinol salts are chosen from those of formula (XV) below: 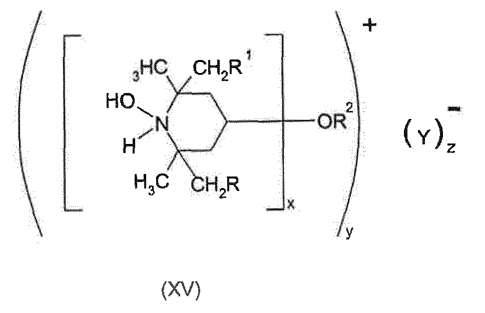 in which
R¹ denotes hydrogen or methyl
x is 1 or 2
1) when x is equal to 1:
R² denotes a hydrogen; a C₁-C₁₈ alkyl radical; a C₂-C₁₈ alkenyl radical; a propargyl radical; a glycidyl group; a C₂-C₅₀ alkyl radical interrupted with 1 to 20 oxygen atoms, the said alkyl being substituted with 1 to 10 hydroxyl groups or alternatively simultaneously interrupted with the said oxygen atoms and substituted with the said hydroxyl groups; a C₁-C₄ alkyl radical substituted with a carboxyl group or a group -COOZ in which Z is hydrogen, a C₁-C₄ alkyl, phenyl, a C₁-C₄ alkyl substituted with a group (COO⁻)ₚ M^{p+} where p is an integer from 1 to 3 and M is a metal ion from groups 1, 2 and 3 of the Periodic Table or Zn, Cu, Ni or Co, or alternatively M is a group N^{p+}(R")₄ where R" is a C₁-C₈ alkyl or a benzyl;
2) when x is 2:
R' is a C₁-C₁₂ alkylene radical; a C₄-C₁₂ alkenylene radical; a xylylene group; a C₁-C₅₀ alkylene radical interrupted with 1 to 20 oxygen atoms, the said alkyl being substituted with 1 to 10 hydroxyl groups or alternatively simultaneously interrupted with the said oxygen atoms and substituted with the said hydroxyl groups;
Y denotes an organic or mineral anion;
the total charge of cations y being equal to the total charge of anions z.

11. Composition according to Claim 10, in which the compounds of formula (XV) are chosen from those for which R¹ and R² denote hydrogen, x = 1 and Y denotes a citrate anion.

12. Composition according to Claim 11, in which the compound of formula (XV) is tris(tetramethyl-hydroxypiperidinol) citrate of structure: 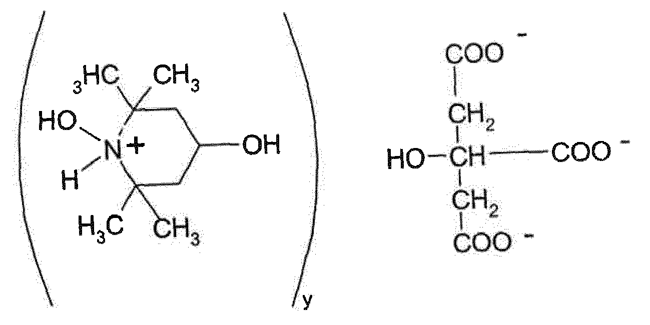 with y = 3.

13. Composition according to any one of Claims 1 to 12, in which the compounds capable of accepting the energy of the triplet state of the dibenzoylmethane derivatives are present in contents ranging from 0.1% to 25% by weight, more preferentially from 0.2% to 10% by weight and even more preferentially from 0.2% to 7% by weight relative to the total weight of the composition.

14. Composition according to any one of Claims 1 to 13, **characterized in that** it also contains other UV-A-active and/or UV-B-active organic or mineral photoprotective agents that are water-soluble or liposoluble or insoluble in the cosmetic solvents commonly used.

15. Composition according to Claim 14, in which the additional organic photoprotective agents are chosen from anthranilates; salicylic derivatives, camphor derivatives; benzophenone derivatives; β,β-diphenylacrylate derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; benzoxazole derivatives; screening polymers and screening silicones; α-alkylstyrene-based dimers derivatives; 4,4-diarylbutadienes, and mixtures thereof.

16. Composition according to Claim 15, **characterized in that** the organic UV-screening agent(s) is (are) chosen from the following compounds:
Homosalate
Ethylhexyl salicylate,
Octocrylene,
Phenylbenzimidazolesulfonic acid,
Benzophenone-3,
Benzophenone-4,
Benzophenone-5,
n-Hexyl 2-(4-diethylamino-2-hydroxybenzoyl)benzoate, 4-Methylbenzylidenecamphor,
Terephthalylidenedicamphorsulfonic acid,
Disodium phenyldibenzimidazoletetrasulfonate, Methylenebis(benzotriazolyl)tetramethylbutylphenol,
Ethylhexyl Triazone,
Bis(ethylhexyloxyphenol)methoxyphenyltriazine Diethylhexyl Butamido Triazone,
2,4,6-Tris(dineopentyl 4'-aminobenzalmalonate)-s-triazine
2,4,6-Tris(diisobutyl 4'-aminobenzalmalonate)-s-triazine,
2,4,6-Tris(biphenyl-4-yl)-1,3,5-triazine, 2,4,6-Tris(terphenyl)-1,3,5-triazine,
Drometrizole trisiloxane,
Polysilicone-15,
Dineopentyl 4-methoxybenzalmalonate,
1,1-Dicarboxy(2,2'-dimethylpropyl)-4,4-diphenyl-butadiene,
2,4-Bis[5-1(dimethylpropyl)benzoxazol-2-yl(4-phenyl)-imino]-6-(2-ethylhexyl)imino-1,3,5-triazine,
and mixtures thereof.

17. Composition according to Claim 14, **characterized in that** the additional mineral photoprotective agents are treated or untreated metal oxide pigments or nanopigments.

18. Composition according to Claim 17, **characterized in that** the said pigments or nanopigments are chosen from titanium oxide, zinc oxide, iron oxide, zirconium oxide and cerium oxide, and mixtures thereof, which are treated or untreated.

19. Composition according to any one of Claims 1 to 18, **characterized in that** it also comprises at least one agent for artificially tanning and/or browning the skin.

20. Composition according to any one of Claims 1 to 19, **characterized in that** it also comprises at least one adjuvant chosen from fatty substances, organic solvents, ionic or nonionic, hydrophilic or lipophilic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants and acidifying or basifying agents.

21. Use of a composition as defined in any one of Claims 1 to 20, for the manufacture of products for cosmetic treatment of the skin, the lips, the nails, the hair, the eyelashes, the eyebrows and/or the scalp.

22. Use of a composition as defined in any one of Claims 1 to 20, for the manufacture of care products for the skin, the lips, the nails, the hair and/or the scalp.

23. Use of a composition as defined in any one of Claims 1 to 20, for the manufacture of makeup products.

24. Process for photostabilizing a dibenzoylmethane derivative as defined in any one of the preceding claims against UV radiation via a combination of 2-phenylethyl benzoate and a compound capable of accepting the excited triplet level energy of the said dibenzoylmethane derivative as defined in any one of the preceding claims.

25. Use of a combination of 2-phenyethyl benzoate and a compound capable of accepting the excited triplet level energy as defined in any one of the preceding claims, in a cosmetic or dermatological composition comprising at least one dibenzoylmethane derivative as defined in any one of the preceding claims, for the purpose of improving the stability towards UV rays of the said dibenzoylmethane derivative.

## Patentansprüche

1. Lichtschutzzusammensetzung zur topischen Verwendung, **dadurch gekennzeichnet, dass** sie in einem kosmetisch unbedenklichen Träger mindestens:
(a) mindestens ein UV-Filter vom Dibenzoylmethanderivat-Typ und
(b) Benzoesäure-2-phenylethylester der Formel: 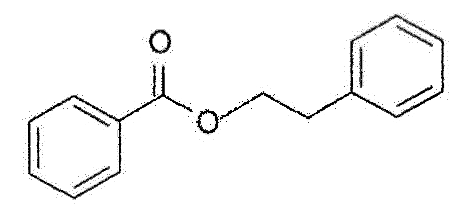
(c) mindestens eine Verbindung, die die Energie des angeregten Triplettzustands des Dibenzoylmethanderivats aufnehmen kann, ausgewählt aus
i. 4-Hydroxybenzylidenmalonatderivaten oder 4-Hydroxycinnamatderivaten;
ii. Piperidinolsalzen;
iii. Mischungen davon;
umfasst.

2. Zusammensetzung nach Anspruch 1, wobei das Dibenzoylmethanderivat aus
- 2-Methyldibenzoylmethan,
- 4-Methyldibenzoylmethan,
- 4-Isopropyldibenzoylmethan,
- 4-tert.-Butyldibenzoylmethan,
- 2,4-Dimethyldibenzoylmethan,
- 2,5-Dimethyldibenzoylmethan,
- 4,4'-Diisopropyldibenzoylmethan,
- 4,4'-Dimethoxydibenzoylmethan,
- 4-tert.-Butyl-4'-methoxydibenzoylmethan,
- 2-Methyl-5-isopropyl-4'-methoxydibenzoylmethan,
- 2-Methyl-5-tert.-butyl-4'-methoxydibenzoylmethan,
- 2,4-Dimethyl-4'-methoxydibenzoylmethan und
- 2,6-Dimethyl-4-tert.-butyl-4'-methoxydibenzoylmethan
ausgewählt ist.

3. Zusammensetzung nach Anspruch 1 oder 2, wobei es sich bei dem Dibenzoylmethanderivat um 4-(tert.-Butyl)-4'-methoxydibenzoylmethan oder Butylmethoxydibenzoylmethan handelt.

4. Zusammensetzung nach einem der Ansprüche 1 bis 3, wobei der Benzoesäure-2-phenylethylester in Gehalten von 0,1 bis 40 Gew.-% und weiter bevorzugt 1 bis 30 Gew.-%, bezogen auf das Gesamtgewicht der Zusammensetzung, vorliegt.

5. Zusammensetzung nach einem der Ansprüche 1 bis 4, wobei das Dibenzoylmethanderivat bzw. die Dibenzoylmethanderivate in Gehalten von 0,01 bis 10 Gew.-% und weiter bevorzugt 0,1 bis 6 Gew.-%, bezogen auf das Gesamtgewicht der Zusammensetzung, vorliegt bzw. vorliegen.

6. Zusammensetzung nach einem der Ansprüche 1 bis 5, wobei die 4-Hydroxybenzylidenmalonatderivate oder 4-Hydroxycinnamatderivate aus denjenigen der Formel (VI) ausgewählt sind: 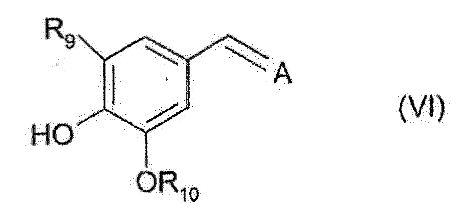 worin A für eine UV-Strahlung absorbierende chromophore Gruppe mit zwei einwertigen Gruppen mit einer Carbonylfunktion steht;
R₉ für Wasserstoff, einen linearen oder verzweigten C₁-C₈-Alkylrest oder einen linearen oder verzweigten C₁-C₈-Alkoxyrest steht;
R₁₀ für einen linearen oder verzweigten C₁-C₈-Alkylrest steht.

7. Zusammensetzung nach Anspruch 6, wobei die Verbildungen der Formel (VI) aus denjenigen der folgenden Formel (VIa) ausgewählt sind: 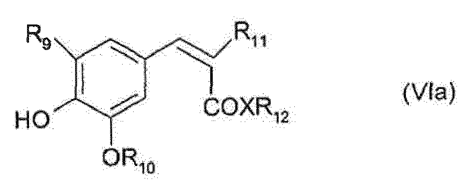 worin R₁₁ aus -C(O)CH₃, -CO₂R₁₃, -C(O)NH₂ und - C(O)N(R₁₄)₂ ausgewählt ist;
X für O oder NH steht;
R₁₂ für einen linearen oder verzweigten C₁-C₃₀-Alkylrest steht;
R₁₃ für einen linearen oder verzweigten C₁-C₂₀-Alkylrest steht;
R₁₄ jeweils unabhängig voneinander für einen linearen oder verzweigten C₁-C₈-Alkylrest steht;
Rg für Wasserstoff, linearen oder verzweigten C₁-C₈-Alkylrest oder einen linearen oder verzweigten C₁-C₈-Alkoxyrest steht;
R₁₀ für Wasserstoff, einen linearen oder verzweigten C₁-C₈-Alkylrest steht.

8. Zusammensetzung nach Anspruch 6, wobei die Verbindungen der Formel (VI) aus denjenigen der folgenden Formel (VIb) oder (VIc) ausgewählt sind: 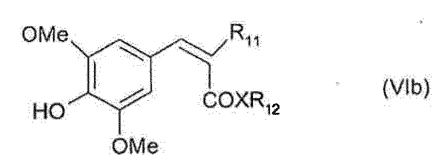 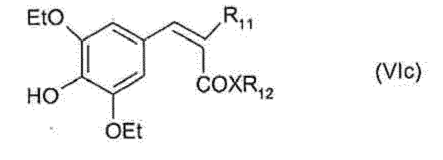 worin
R₁₁ für -CO₂R₁₃ steht;
R₁₂ für einen linearen oder verzweigten C₁-C₈-Alkylrest steht;
R₁₃ für einen linearen oder verzweigten C₁-C₈-Alkylrest steht:
X für O steht.

9. Zuaammensetzung nach Anspruch 8, wobei es sich bei der Verbindung der Formel (VIb) zum Diethylhexyl Syringylidene Malonate der folgenden Formel handelt:

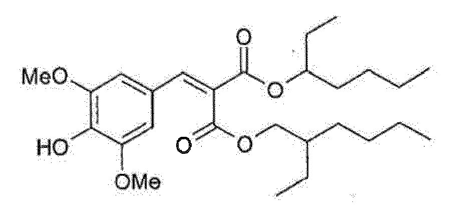

10. Zusammensetzung nach einem der Ansprüche 1 bis 5, wobei die Piperidinolsalze aus denjenigen der folgendem Formel (XV) ausgewählt sind: 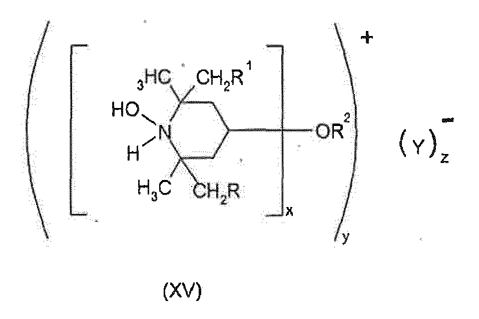 worin
R¹ für Wasserstoff oder Methyl steht,
x für 1 oder 2 steht,
1) wenn x gleich 1 ist:
R² für Wasserstoff; einen C₁-C₁₈-Alkylrest; einen C₂-C₁₈-Alkenylrest; einen Propargylrest; eine Glycidylgruppe; einen C₂-C₅₀-Alkylrest, der durch 1 bis 20 Sauerstoffatome unterbrochen ist, wobei das Alkyl durch 1 bis 10 Hydroxylgruppen substituiert oder auch gleichzeitig durch die Sauerstoffatome unterbrochen und durch die Hydroxylgruppen substituiert ist; oder einen C₁-C₄-Alkylrest, der durch eine Carboxygruppe oder eine -COOZ-Gruppe, worin Z für Wasserstoff, ein C₁-C₄-Alkyl, Phenyl oder ein C₁-C₄-Alkyl, das durch eine Gruppe (COO⁻)ₚM^{p+}, worin p für eine ganze Zahl von 1 bis 3 steht und M für ein Metallion der Gruppen 1, 2 und 3 des Periodensystems oder von Zn, Cu, Ni oder Co steht oder M auch für eine Gruppe N^{p+}(R")₄ steht, worin R" für ein C₁-C₈-Alkyl oder ein Benzyl steht, substituiert ist, steht, substituiert ist, steht;
2) wenn x gleich 2 ist:
R¹ für einen C₁-C₁₂-Alkylenrest; einen C₄-C₁₂-Alkenylenrest; eine Xylylengruppe oder einen C₁-C₅₀-Alkylenrest, der durch 1 bis 20 Sauerstoffatome unterbrochen ist, wobei das Alkyl durch 1 bis 10 Hydroxylgruppen substituiert oder auch gleichzeitig durch die Sauerstoffatome unterbrochen und durch die Hydroxylgruppen substituiert ist; steht;
Y für ein organisches oder mineralisches Anion steht;
wobei die Kationengesamtladung y gleich der Anionengesamtladung z ist.

11. Zusammensetzung nach Anspruch 10, wobei die Verbindungen der Formel (XV) aus denjenigen ausgewählt sind, für die R¹ und R² für Wasserstoff stehen, x = 1 und Y für das Citrat-Anion stehen.

12. Zusammensetzung nach Anspruch 11, wobei es sich bei der Verbindung der Formel (XV) um Tris(tetramethylhydroxypiperidinol)-citrat der Struktur: 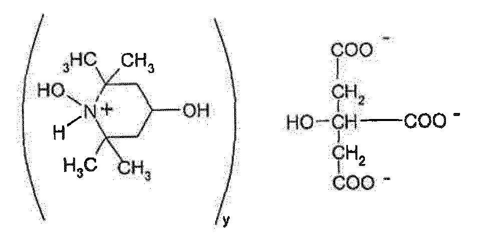 mit y = 3 handelt.

13. Zusammensetzung nach einem der Ansprüche 1 bis 12, wobei die Verbindungen, die die Energie des Triplettzustands der Dibenzoylmethanderivate aufnehmen können, in Gehalten von 0,1 bis 25 Gew.-% und weiter bevorzugt 0,2 bis 10 Gew.-% und noch weiter bevorzugt 0,2 bis 7 Gew.-%, bezogen auf das Gesamtgewicht der Zusammensetzung, vorliegen.

14. Zusammensetzung nach einem der Ansprüche 1 bis 13, **dadurch gekennzeichnet, dass** sie außerdem andere UV-A- und/oder UV-B-aktive organische oder anorganische Lichtschutzmittel, die wasserlöslich oder fettlöslich oder auch in den gemeinhin verwendeten kosmetischen Lösungsmitteln unlöslich sind, enthält.

15. Zusammensetzung nach Anspruch 14, wobei die zusätzlichen organischen Lichtschutzmittel aus Anthranilaten; Salicylsäurederivaten, Campherderivaten; Benzophenonderivaten; β,β-Diphenylacrylatderivaten; Benzotriazolderivaten, Benzalmalonatderivaten; Benzimidazolderivaten; Imidazolinen; Bisbenzoazolylderivaten; p-Aminobenzoesäurederivaten (PABA-Derivaten); Methylenbis(hydroxyphenylbenzotriazol)derivaten; Benzoxazolderivaten; Filterpolymeren und Filtersilikonen; von α-Alkylstyrol abgeleiteten Dimeren; 4,4-Diarylbutadienen und Mischungen davon ausgewählt sind.

16. Zusammensetzung nach Anspruch 15, **dadurch gekennzeichnet, dass** das organische UV-Filter bzw. die organischen UV-Filter aus den folgenden Verbindungen ausgewählt ist bzw. sind:
Homosalate,
Ethylhexyl Salicylate,
Octocrylene,
Phenylbenzimidazole Sulfonic Acid,
Benzophenone-3,
Benzophenone-4,
Benzophenone-5,
2-(4-Diethylamino-2-hydroxybenzoyl)benzoesäure-n-hexylester,
4-Methylbenzylidencampher,
Terephthalylidene Dicamphor Sulfonic Acid,
Disodium Phenyl Dibenzimidazole Tetrasulfonate,
Methylenbis(benzotriazolyl)tetramethylbutylphenol, Ethylhexyltriazon,
Bis-Ethylhexyloxyphenol Methoxyphenyl Triazine, Diethylhexyl Butamido Triazone,
2,4,6-Tris(4'-aminobenzalmalonsäuredineopentylester)-s-triazin,
2,4,6-Tris(4'-aminobenzalmalonsäurediisobutylester)-s-triazin,
2,4,6-Tris(biphenyl-4-yl)-1,3,5-triazin,
2,4,6-Tris(terphenyl)-1,3,5-triazin,
Drometrizole Trisiloxane,
Polysilicone-15,
Dineopentyl 4'-Methoxybenzalmalonate,
1,1-Dicarboxy(2,2'-dimethylpropyl)-4,4-diphenylbutadien,
2,4-Bis[5-1(dimethylpropyl)benzoxazol-2-yl(4-phenyl)imino]-6-(2-ethylhexyl)imino-1,3,5-triazin und Mischungen davon.

17. Zusammensetzung nach Anspruch 14, **dadurch gekennzeichnet, dass** es sich bei den zusätzlichen anorganischen Lichtschutzmitteln um behandelte oder unbehandelte Metalloxidpigmente oder -nanopigmente handelt.

18. Zusammensetzung nach Anspruch 17, **dadurch gekennzeichnet, dass** die Pigmente oder Nanopigmente aus Oxiden von Titan, Zink, Eisen, Zirconium, Cer und Mischungen davon, die behandelt oder unbehandelt sein können, ausgewählt sind.

19. Zusammensetzung nach einem der Ansprüche 1 bis 18, **dadurch gekennzeichnet, dass** sie außerdem mindestens ein Mittel zur künstlichen Bräunung und/oder Braunfärbung der Haut umfasst.

20. Zusammensetzung nach einem der Ansprüche 1 bis 19, **dadurch gekennzeichnet, dass** sie außerdem mindestens einen Hilfsstoff, der unter Fettsubstanzen, organischen Lösungsmitteln, ionischen oder nichtionischen, hydrophilen oder lipophilen Verdickungsmitteln, zartmachenden Mitteln, Feuchtigkeitsspendern, Trübungsmitteln, Stabilisatoren, Emollientien, Silikonen, Schaumhemmern, Duftstoffen, Konservierungsmitteln, anionischen, kationischen, nichtionischen, zwitterionischen oder amphoteren Tensiden, Wirkstoffen, Füllstoffen, Polymeren, Treibmitteln, Alkalinisierungsmitteln oder Ansäuerungsmitteln ausgewählt ist, umfasst.

21. Verwendung einer Zusammensetzung gemäß einem der Ansprüche 1 bis 20 zur Herstellung von Produkten für die kosmetische Behandlung der Haut, der Lippen, der Nägel, der Haare, der Wimpern, der Augenbrauen und/oder der Kopfhaut.

22. Verwendung einer Zusammensetzung gemäß einem der Ansprüche 1 bis 20 zur Herstellung von Produkten für die Pflege der Haut, der Lippen, der Nägel, der Haare und/oder der Kopfhaut.

23. Verwendung einer Zusammensetzung gemäß einem der Ansprüche 1 bis 20 zur Herstellung von Makeup-Produkten.

24. Verfahren zur Photostabilisierung eines Dibenzoylmethanderivats gemäß einem der vorhergehenden Ansprüche gegenüber UV-Strahlung durch die Kombination von Benzoesäure-2-phenylethylester und einer Verbindung, die die Energie des angeregten Triplettzustands des Dibenzoylmethanderivats aufnehmen kann, gemäß einem der vorhergehenden Ansprüche.

25. Verwendung der Kombination von Benzoesäure-2-phenylethylester und einer Verbindung, die die Energie des angeregten Triplettzustands eines Dibenzoylmethanderivats aufnehmen kann, gemäß einem der vorhergehenden Ansprüche in einer kosmetischen oder dermatologischen Zusammensetzung, die mindestens ein Dibenzoylmethanderivat gemäß einem der vorhergehenden Ansprüche umfasst, zur Verbesserung der Stabilität des Dibenzoylmethanderivats gegenüber UV-Strahlen.
